# FASCICULE DE BREVET EUROPEEN

(11) **EP 2 274 097 B1**
(45) Date de publication et mention de la délivrance du brevet: **08.07.2020**
(21) Numéro de dépôt: 09723477.7
(22) Date de dépôt: 10.03.2009
(51) Int. Cl.: B01L 3/00, A01N 1/00

(54) **CONTENANT DESTINE A RECEVOIR ET CONSERVER DU MATERIEL BIOLOGIQUE, NOTAMMENT DE L'ADN**
BEHÄLTER ZUR AUFNAHME UND LAGERUNG VON BIOLOGISCHEM MATERIAL, INSBESONDERE DNA
CONTAINER FOR RECEIVING AND STORING BIOLOGICAL MATERIAL, ESPECIALLY DNA

(30) Priorité: 11.03.2008 FR 0851562
(43) Date de publication de la demande: 19.01.2011
(73) Titulaire: Imagene, 33600 Pessac (FR)
(72) Inventeur: TUFFET, Sophie, F-33800 Bordeaux (FR); DE SOUZA, David Georges, F-33800 Bordeaux (FR)
(74) Mandataire: Aquinov
(86) Numéro de dépôt international: PCT/FR2009/050393
(87) Numéro de publication internationale: WO 2009/115760

(56) Documents cités:
- EP-A- 0 040 014
- EP-A- 0 200 098
- DE-A1- 4 314 137
- JP-A- 9 174 269
- US-A- 3 272 383
- US-A- 3 355 060
- US-A- 4 821 914
- US-A1- 2007 116 613

## Description

La présente invention concerne un contenant destiné à recevoir et conserver du matériel biologique, notamment de l'ADN.

L'ADN nécessite sa conservation sur de très longues durées tant dans le domaine de la recherche que dans de très nombreux autres domaines comme les biotechnologies, la santé, l'environnement, l'agroalimentaire, l'identification, la justice, la criminalistique, par exemple et notamment en vue de la réalisation de bibliothèques d'échantillons biologiques ou biothèques.

Le matériel biologique est d'origine humaine, animale ou végétale et comprend notamment : les tissus ; les cellules ; les microorganismes tels que bactéries, champignons, algues monocellulaires ; les virus ; les protéines ; les acides nucléiques tels ADN, ARN.

Le problème est de pouvoir conserver ce matériel biologique dont les éléments dégradants sont l'oxygène de l'air, l'eau et la lumière. Il convient également de protéger ce matériel biologique de tout élément contaminant.

On connaît le brevet EP 1 075 515 qui prévoit un procédé de conservation de l'ADN de longue durée dans une capsule métallique, inoxydable et étanche, constituée de deux hémisphères dans son mode de réalisation envisagé. Le document US 4,821,914 divulgue également un contenant métallique destiné, entre autres, à la conservation de matériau biologique.

Cet ADN est encapsulé en atmosphère neutre et avec un très faible degré hygrométrique afin d'en permettre la conservation à température ambiante, évitant donc la mise en œuvre de moyens de réfrigération.

Si ces moyens de conservation sous forme de capsule sont satisfaisants, ils restent difficilement industrialisables.

Il convient en effet de pouvoir réutiliser l'ADN contenu dans la capsule de façon aisée, voire de pouvoir la réutiliser plusieurs fois.

De plus, dans le domaine biologique il est nécessaire de prévoir une étape d'aliquotage pour réaliser une multiplicité d'échantillons.

Or une capsule du type double hémisphères n'est pas le moyen le plus adéquat et c'est pour cette raison que la présente invention propose un contenant destiné à recevoir du matériel biologique et plus particulièrement de l'ADN, qui peut être obturé de façon totalement étanche, qui permet une ouverture sans dégradation dudit contenu et qui permet une conservation et plusieurs réutilisations successives de l'ADN reconstitué. De plus, il est nécessaire de prévoir une identification de chacune des capsules, identification qui doit être pérenne, y compris après ouverture dudit contenant.

Le contenant selon la présente invention est défini à la revendication 1 et est décrit en détail selon un mode de réalisation préférentiel mais non limitatif, les dessins annexés permettant une illustration de l'invention, les différentes figures représentant :
- figure 1 : une vue en perspective du contenant avant sa mise en service,
- figure 2 : une vue en perspective du contenant assemblé et en service avant soudure,
- figure 3 : une vue en perspective du contenant assemblé et en service après soudure,
- figure 4 : une vue de dessous du contenant après marquage,
- figure 5 : une vue en perspective du contenant après ouverture pour réutilisations avec un obturateur.

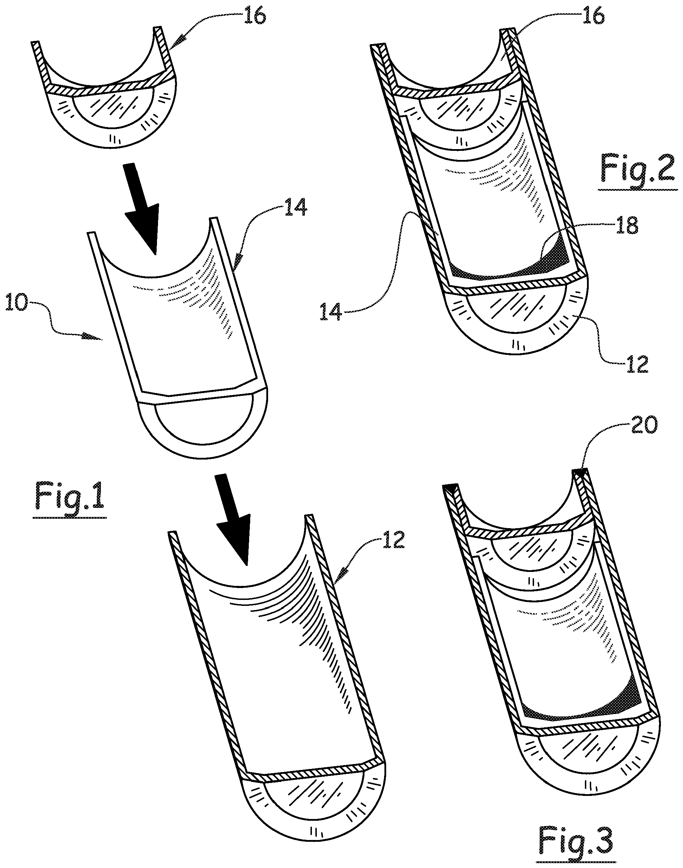

Sur la figure 1, on a représenté un contenant 10 de forme cylindrique et pour le mode de réalisation préférentiel de 7 mm de diamètre et de 18 mm de hauteur pour une épaisseur de quelques dixièmes de millimètres.

Ce contenant est composé d'une enveloppe 12, fermée à une extrémité, qui doit être réalisée en un matériau imperméable aux gaz, résistant à la corrosion, apte à être obturé de façon étanche.

Dans le cas présent, le matériau doit aussi répondre à une contrainte supplémentaire qui est celle de la déformabilité et donc de sa malléabilité pour réaliser ledit contenant de forme cylindrique fermée à une extrémité.

En effet, pour réaliser l'enveloppe, l'emboutissage profond est la solution la plus adaptée industriellement d'une part pour respecter la précision dimensionnelle nécessaire comme cela sera expliqué plus avant et d'autre part pour rester dans des conditions de coût en adéquation avec un usage en très grandes séries.

De fait, la nuance d'acier inoxydable adaptée et préférée pour la réalisation de l'enveloppe selon la présente invention est connu commercialement sous la référence 304L ou plus métallurgiquement sous la nuance Z2CN18-10.

Le contenant 10 est complété par un insert 14, également cylindrique et venant se loger à frottement doux dans l'enveloppe 12.

Cet insert est avantageusement réalisé en verre pour ses aptitudes à conserver le matériel biologique et pour sa stabilité.

L'insert, ainsi que représenté, a une hauteur inférieure à celle de l'enveloppe.

Le contenant 10 comporte aussi un bouchon 16 destiné à venir s'emboîter en force dans l'extrémité ouverte de l'enveloppe cylindrique.

Ce bouchon 16 est également de forme cylindrique, son diamètre extérieur étant égal, aux frottements nécessaires près, pour entrer en force dans l'enveloppe cylindrique.

Le bouchon est avantageusement obtenu par déformation mécanique du type emboutissage comme l'enveloppe et pour les mêmes raisons.

La hauteur de ce bouchon cylindrique est faible, de l'ordre de quelques millimètres en l'occurrence 3 mm, comparés aux 18 mm environ de l'enveloppe cylindrique. L'épaisseur du bouchon est également de quelques dixièmes de millimètres, préférentiellement identique ou très voisine de celle de l'enveloppe en l'occurrence 0,25 mm, ceci pour des raisons de paramètres de soudage, comme il sera expliqué plus avant.

Le bouchon est réalisé dans le même matériau que l'enveloppe, pour disposer au final d'une enveloppe de contenant monolithique et monomatière.

On note sur la figure 2 que l'enveloppe ayant reçue le bouchon conduit à une géométrie telle que les bords périphériques ouverts de l'enveloppe et du bouchon se trouvent juxtaposés.

Avantageusement, l'enveloppe comme le bouchon comprennent un chanfrein ou un congé périphérique à l'extrémité, résultant de la déformation par emboutissage mais permettant aussi une parfaite introduction du bouchon dans l'enveloppe.

Selon un perfectionnement de l'invention, il est possible de prévoir un bouchon avec une légère conicité de façon à provoquer son immobilisation en translation après introduction.

Sur la figure 2, on a représenté de façon schématique le matériel biologique 18, déposé dans l'insert 14, ledit matériel biologique étant à l'état déshydraté. Avantageusement, il est prévu d'introduire une atmosphère contrôlée dans le contenant fermé et devant contenir et conserver ledit matériel biologique.

Sur la figure 3, on a représenté le contenant scellé de façon hermétique et définitive.

Un moyen adapté est une soudure 20 du bouchon sur l'enveloppe.

C'est là que le contenant selon l'invention montre encore son intérêt. En effet, le matériel biologique étant introduit préalablement à la réalisation de la soudure et pour éviter toute dégradation ou tout endommagement de ce matériel biologique, il est impératif de limiter voire d'éviter tout échauffement.

De fait l'agencement selon l'invention prévoit un soudage par laser appliqué de façon périphérique sur les deux bords de l'enveloppe et du bouchon.

Un tel soudage ne nécessite aucun métal d'apport et ne modifie pas la structure du matériau qui conserve toutes ses propriétés initiales.

L'épaisseur identique ou très voisine des deux matériaux est également un avantage pour obtenir une réalisation homogène de la soudure.

Ce soudage nécessite un tir laser de très faible puissance eu égard à la très faible épaisseur des parois si bien qu'il ne se produit aucune élévation significative de la température de la paroi et/ou dans l'enceinte et dans tous les cas totalement non susceptible de provoquer une modification et/ou une dégradation du matériel biologique.

Avantageusement, on recourt à un laser de type Y AG pulsé si bien que l'élévation de température est de façon avérée négligeable.

Il est également à noter que la soudure a uniquement un rôle d'étanchéité et un rôle d'immobilisation en translation du bouchon dans l'enveloppe sans nécessiter des capacités importantes de résistance mécanique de liaison.

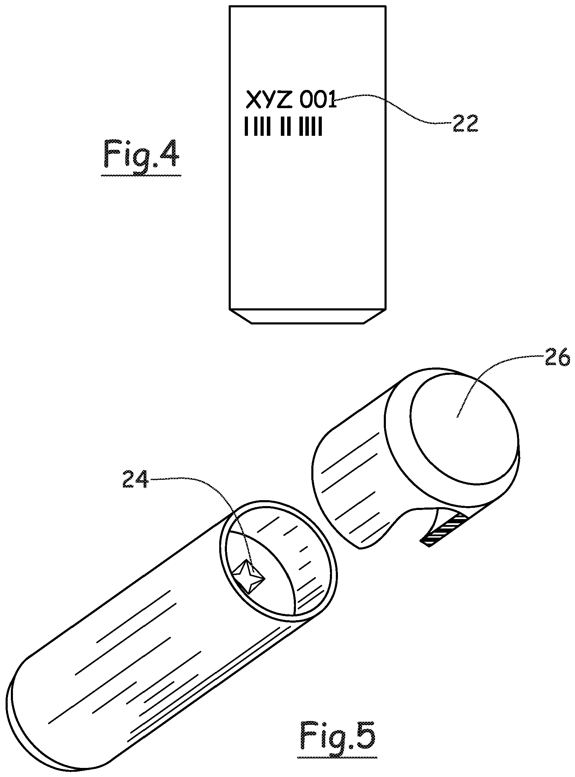

Sur la figure 4, on a représenté un marquage qui est généralement réalisé préalablement à l'introduction du matériel biologique pour un respect des procédures de traçabilité.

De plus, ce marquage doit être aussi pérenne que le contenant, ce qui incite à éviter des éléments rapportés et susceptibles d'être rapidement et aisément dégradés comme un marquage peinture, une étiquette, une impression par exemple.

Le marquage du contenant selon la présente invention est obtenu par réalisation d'une marque 22. Cette marque elle-même peut contenir tout type de référence chiffres, lettres ou codes à barres ou encore codes matriciels du type Data Matrix.

Avantageusement, le marquage est obtenu par changement de l'état de surface de la matière sous l'effet d'un faisceau laser adapté. Dans ce cas, la matière n'est pas gravée en creux, le marquage étant néanmoins pérenne.

On constate là aussi que le marquage au moyen d'un faisceau laser engendre une dissipation d'énergie totalement négligeable sur le contenant, y compris lorsque le marquage est réalisé sur le fond du contenant.

Un autre moyen est celui de rapporter une étiquette de type RFID, c'est-à-dire une étiquette d'identification utilisant les hyperfréquences. Dans ce cas, l'étiquette a pour rôle de plaquer et de maintenir mécaniquement une antenne sur la paroi du contenant, cet antenne étant l'élément actif. L'étiquette ne porte pas elle-même une impression et un tel moyen est considéré comme pérenne au sens de l'invention. Toute dégradation superficielle de l'étiquette ne porte aucunement atteinte à l'élément actif d'indentification.

Si l'on se reporte à la figure 5, on constate que le contenant permet une utilisation du matériel biologique contenu et conservé.

Ainsi, il est prévu de ménager une fenêtre 24 d'accès, plus particulièrement par découpage perforant à l'aide d'un outil par exemple un poinçon en forme de pointe de diamant, au fond du bouchon. La pointe diamant est positionnée au centre par repérage.

La pression à exercer est très faible du fait de l'épaisseur mince du fond du bouchon.

On constate aussi que le poinçonnement conduit à une découpe de plusieurs volets qui, sous l'effet de la mémoire de forme du matériau, se roulent laissant une fenêtre avec des bords sans aspérité donc facilitant l'accès mais surtout le retrait des appareillages destinés à être introduits par ladite fenêtre. En effet les pipettes et autres tubes viennent en appui par des génératrices sur les génératrices des volets.

Surtout le matériel biologique ne peut pas être pollué par des particules métalliques puisqu'il n'y a pas usinage mais seulement découpe par emboutissage de la matière.

On note également que l'insert ne peut pas non plus être dégradé mécaniquement lors de cette opération puisqu'il est périphérique.

Le marquage reste également tout à fait accessible et lisible puisque le contenant ne subit aucune action mécanique seul le fond du bouchon étant soumis à l'action de poinçonnage par la pointe en forme de diamant.

Cette fenêtre 24 permet donc l'introduction d'une pipette, d'une seringue ou d'un instrument analogue pour adjoindre tout liquide adapté pour mettre en solution le matériel biologique déshydraté.

Ce matériel biologique mis en suspension peut alors être retiré en totalité ou partiellement en fonction des besoins avec les mêmes types d'instruments.

Bien entendu, l'ouverture est définitive et le contenant ne peut plus permettre une conservation de très longue durée.

Par contre, le matériel biologique peut n'être utilisé que partiellement et nécessiter une conservation sur quelques jours par exemple pour différentes manipulations.

Dans ce cas, le contenant est également utilisable pour conserver le matériel biologique remis en solution en prenant la précaution de placer sur le contenant un obturateur 26, par exemple en matériau élastomère neutre.

Cet obturateur est soit sous forme de coiffe comme représenté sur la figure 5, soit de forme tronconique pour venir se loger dans le bouchon directement en y étant retenu par les efforts élastiques radiaux.

A noter que le marquage reste associé de façon définitive au contenant et que le repérage du contenant ouvert en vue d'une réutilisation après ouverture reste possible sans induire une quelconque erreur.

On constate aussi que le contenant cylindrique est également parfaitement adapté à des manipulations par des automates et pour un positionnement sur des portoirs à microplaques couramment utilisés en biologie, notamment les portoirs au standard connu SBS, marque déposée.

De même, le marquage gravé est extrêmement bien lisible pour un lecteur optique, sans engendrer d'erreur de lecture du fait d'une éventuelle dégradation de ce marquage comme cela pourrait être le cas avec des étiquettes rapportées par exemple.

La traçabilité des échantillons de matériel biologique peut donc être organisée en recourant au contenant selon la présente invention.

Le contenant permet de bénéficier de tous les avantages liés à la conservation à température ambiante et notamment de faciliter les échanges entre machines d'analyse, entre laboratoires et de réaliser des biothèques mais en plus d'autoriser l'utilisation d'automates pour la mise en place du matériel biologique dans ledit contenant.

Ces étapes comprennent au moins les opérations de déshydratation, d'introduction d'une atmosphère contrôlée, de fermeture par soudage et de marquage.

Même l'ouverture par poinçonnage peut être réalisée à l'aide d'un dispositif équipé d'un poinçon de façon à pouvoir régler les courses et assurer un guidage parfait.

Les automates d'analyse, y compris les automates existants peuvent être munis d'un tel dispositif d'ouverture par poinçonnage, évitant toute intervention humaine.

L'insert a été présenté comme réalisé en verre mais il est aussi possible de prévoir en lieu et place un insert en céramique ou tout autre matériau inerte, connu ou à venir, apte à conserver le matériel biologique.

De même, l'insert peut être remplacé dans certaines applications par des billes de matériau inerte vis-à-vis du matériel biologique et chargées en ce matériel biologique préalablement par adsorption.

Le contenant est prévu pour conserver le matériel biologique à température ambiante mais pour certains matériels biologiques pour lesquels il était nécessaire d'assurer une conservation à une température plus basse ou égale à -20°C, on constate que la température de conservation au sein du contenant selon l'invention peut être ramenée à des températures positives de quelques degrés.

## Revendications

1. Contenant (10) destiné à conserver du matériel biologique déshydraté sous atmosphère contrôlée, notamment à température ambiante et plus particulièrement de l'ADN, comprenant une enveloppe (12) en matériau métallique étanche aux gaz, **caractérisé en ce que** l'enveloppe (12) est de forme cylindrique, le contenant comprenant un insert (14), également de forme cylindrique et venant se loger à frottement doux dans l'enveloppe (12), pour recevoir ledit matériel biologique, ladite enveloppe étant fermée à une extrémité et comprend, à l'autre extrémité, un bouchon (16) destiné à être lié de façon étanche à ladite enveloppe, l'enveloppe (12) et le bouchon (16) étant réalisés dans le même matériau, chacun, monolithique et venu de déformation mécanique par emboutissage profond, le bouchon (16) étant en matériau métallique et de forme cylindrique, fermé à une extrémité et prévu pour être emboîté dans l'enveloppe (12) de forme cylindrique, les bords périphériques ouverts de l'enveloppe et du bouchon se trouvant juxtaposés.

2. Contenant (10) destiné à conserver à température ambiante du matériel biologique, selon la revendication 1, **caractérisé en ce que** la liaison est réalisée au moyen d'une soudure (20) des bords périphériques de l'enveloppe (12) et du bouchon (16).

3. Contenant (10) destiné à conserver à température ambiante du matériel biologique, selon la revendication 2, **caractérisé en ce que** la soudure (20) est réalisée par un faisceau laser.

4. Contenant (10) destiné à conserver à température ambiante du matériel biologique selon l'une quelconque des revendications précédentes, **caractérisé en ce qu'**il présente des dimensions de 7 mm de diamètre et de 18 mm de hauteur pour une épaisseur de quelques centièmes de millimètres, de façon à pouvoir être logé dans un puits de plaques à puits.

5. Contenant (10) destiné à conserver à température ambiante du matériel biologique selon l'une quelconque des revendications précédentes, **caractérisé en ce que** le matériau métallique est de l'acier inoxydable Z2CN18-10.

6. Contenant (10) destiné à conserver à température ambiante du matériel biologique selon l'une quelconque des revendications précédentes, **caractérisé en ce que** le bouchon (16) peut être ouvert par poinçonnage.

7. Contenant (10) destiné à conserver à température ambiante du matériel biologique selon l'une quelconque des revendications précédentes, **caractérisé en ce qu'**il comprend un obturateur (26) destiné à venir obturer l'enveloppe après ouverture du bouchon dudit contenant.

## Patentansprüche

1. Behälter (10) für die Aufbewahrung von unter genau definierter Atmosphäre dehydratisiertem biologischen Material, insbesondere von DNA bei Raumtemperatur, eine gasdichte Metallhülle (12) umfassend, **dadurch gekennzeichnet, dass** die Hülle (12) zylinderförmig ist, der Behälter einen ebenfalls zylinderförmigen Einsatz (14) umfasst, der leicht gleitend in der Hülle (12) gelagert ist, um das biologische Material aufzunehmen, wobei diese Hülle an einem Ende geschlossen ist und am anderen Ende einen Deckel (16) umfasst, der dichtend mit dieser Hülle verbunden ist, wobei die Hülle (12) und der Deckel (16) jeweils aus dem gleichen Material und nach einem monolithischen Konzept durch Tiefziehen mechanisch verformt hergestellt sind, wobei der Deckel (16) aus einem metallischen Material, zylinderförmig und an einem Ende geschlossen und dazu vorgesehen ist, in der ebenfalls zylinderförmigen Hülle (12) eingefügt zu werden, wobei die offenen peripheren Ränder der Hülle und des Deckels nebeneinander liegen.

2. Behälter (10) zur Aufbewahrung von biologischem Material bei Raumtemperatur nach Anspruch 1, **dadurch gekennzeichnet, dass** die Verbindung durch eine Schweißnaht (20) der peripheren Ränder von Hülle (12) und Deckel (16) hergestellt wird.

3. Behälter (10) zur Aufbewahrung von biologischem Material bei Raumtemperatur nach Anspruch 2, **dadurch gekennzeichnet, dass** die Schweißnaht (20) durch einen Laserstrahl realisiert wird.

4. Behälter (10) zur Aufbewahrung von biologischem Material bei Raumtemperatur nach einem der vorausgehenden Ansprüche, **dadurch gekennzeichnet, dass** er Maße von 7 mm Durchmesser und 18 mm Höhe bei einer Dicke von einigen hundertstel Millimetern aufweist, sodass er in einem Well von Labor-Mikroplatten aufgenommen werden kann.

5. Behälter (10) zur Aufbewahrung von biologischem Material bei Raumtemperatur nach einem der vorausgehenden Ansprüche, **dadurch gekennzeichnet, dass** es sich bei dem metallischen Material um rostfreien Stahl Z2CN18-10 handelt.

6. Behälter (10) zur Aufbewahrung von biologischem Material bei Raumtemperatur nach einem der vorausgehenden Ansprüche, **dadurch gekennzeichnet, dass** der Deckel (16) durch Lochstanzen geöffnet werden kann.

7. Behälter (10) zur Aufbewahrung von biologischem Material bei Raumtemperatur nach einem der vorausgehenden Ansprüche, **dadurch gekennzeichnet, dass** er eine Verschlusskappe (26) umfasst, die dazu bestimmt ist, die Hülle nach Öffnen des Behälterdeckels abzuschließen.

## Claims

1. A container (10) for storing dehydrated biological material under a controlled atmosphere, in particular at ambient temperature, and more particularly DNA, comprising an envelope (12) made from gastight metal material, **characterised in that** the envelope (12) is cylindrical in shape, the container comprising an insert (14), also cylindrical in shape and being housed with gentle friction in the envelope (12), for receiving said biological material, said envelope being closed at one end and at the other end comprises a stopper (16) intended to be sealingly connected to said envelope, the envelope (12) and the stopper (16) being produced from the same material, each in a single piece and made by mechanical deformation by deep drawing, the stopper (16) being made from metal material and cylindrical in shape, closed at one end and intended to be fitted in the cylindrically shaped envelope (12), the open peripheral edges of the envelope and of the stopper being juxtaposed.

2. A container (10) for storing biological material at ambient temperature, according to claim 1, **characterised in that** the connection is achieved by means of welding (20) of the peripheral edges of the envelope (12) and of the stopper (16).

3. A container (10) for storing biological material at ambient temperature, according to claim 2, **characterised in that** the welding (20) is carried out by a laser beam.

4. A container (10) for storing biological material at ambient temperature according to any one of the preceding claims, **characterised in that** it has dimensions of 7 mm in diameter and 18 mm in height with a thickness of a few hundredths of a millimetre, so as to be able to housed in a well of a well plate.

5. A container (10) for storing biological material at ambient temperature according to any one of the preceding claims, **characterised in that** the metal material is Z2CN18-10 stainless steel.

6. A container (10) for storing biological material at ambient temperature according to any one of the preceding claims, **characterised in that** the stopper (16) can be opened by punching.

7. A container (10) for storing biological material at ambient temperature according to any one of the preceding claims, **characterised in that** it comprises an obturator (26) for closing off the envelope after the stopper of said container is opened.
